(19) Europäisches Patentamt
European Patent Office
Office européen des brevets

(11) Veröffentlichungsnummer: **0 012 321**
**B1**

(12) # EUROPÄISCHE PATENTSCHRIFT

(45) Veröffentlichungstag der Patentschrift: **12.08.81**

(51) Int. Cl.³: **C 07 C 53/02**, C 07 C 51/09, C 07 C 51/48, C 07 C 51/44

(21) Anmeldenummer: **79104857.2**

(22) Anmeldetag: **04.12.79**

(54) **Verfahren zur Gewinnung von wasserfreier oder weitgehend wasserfreier Ameisensäure.**

(30) Priorität: **14.12.78 DE 2853991**

(43) Veröffentlichungstag der Anmeldung: **25.06.80 Patentblatt 80/13**

(45) Bekanntmachung des Hinweises auf die Patenterteilung: **12.08.81 Patentblatt 81/32**

(84) Benannte Vertragsstaaten: **AT BE DE FR GB IT NL SE**

(56) Entgegenhaltungen:
**DE-A-2 407 157**
**DE-A-2 545 658**
**DE-A-2 545 730**
**DE-A-2 744 313**
**CHEMICAL ABSTRACTS, Band 89, Nr. 23, 4. Dezember 1978, Zusammenfassung Nr. 196976c, Seite 578, Columbus, Ohio, US**

(73) Patentinhaber: **BASF Aktiengesellschaft, Carl-Bosch-Strasse 38, D-6700 Ludwigshafen (DE)**

(72) Erfinder: **Bott, Kaspar, Dr. Dipl.-Chem., Rieslingweg 4, D-6706 Wachenheim (DE)**
Erfinder: **Kaibel, Gerd, Wormser Strasse 78, D-6840 Lampertheim (DE)**
Erfinder: **Hoffmann, Herwig, Dr. Dipl.-Chem., Knietschstrasse 21, D-6710 Frankenthal (DE)**
Erfinder: **Kratzer, Otto, Dr. Dipl.-Chem., An der Tuchbleiche 7, D-6712 Bobenheim-Roxheim 2 (DE)**
Erfinder: **Irnich, Rudolf, Dr. Dipl.-Chem., In den Hahndornen 2, D-6719 Bobenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 012 321 B1

# Verfahren zur Gewinnung von wasserfreier oder weitgehend wasserfreier Ameisensäure

Die vorliegende Erfindung betrifft ein neues Verfahren zur Gewinnung von wasserfreier oder weitgehend wasserfreier Ameisensäure durch Hydrolyse von Methylformiat.

Aus »Ullmanns Enzyklopädie der Technischen Chemie«, 4. Auflage, Band 7, Seite 365, ist es bekannt, Ameisensäure durch Acidolyse von Formamid mit Schwefelsäure herzustellen. Dieses im großtechnischen Maßstab ausgeübte Verfahren hat jedoch den Nachteil, daß man hierbei stöchiometrische Mengen an Ammoniumsulfat als Zwangsanfall erhält.

Trotz dieses Nachteils hat die ebenfalls bekannte (Ullmann cit., Seite 366) auf den ersten Blick wesentlich günstiger erscheinende Hydrolyse von Methylformiat

$$HCOOCH_3 + H_2O \rightleftharpoons HCOOH + CH_3OH$$

bisher keinen Eingang in die Technik gefunden, und zwar hauptsächlich wegen der hohen Geschwindigkeit der Rückveresterung, welche durch die katalytisch wirkende starke Ameisensäure bedingt wird. Zwar kann man die Rückveresterung nach dem speziellen Destillationsverfahren der DE-PS 24 07 157 weitgehend unterdrücken, jedoch erfordert dieses Verfahren etwa 7 bis 8 t Dampf pro Tonne Ameisensäure, so daß es allein aus diesem Grunde wirtschaftlich kaum mehr in Betracht kommt. Außerdem erhält man nur das Ameisensäure/Wasser-Azeotrop, also lediglich eine rund 75gew.-%ige Säure, für die im Gegensatz zur reinen oder hochkonzentrierten Säure nur wenig Bedarf besteht.

Weiterhin ist es aus der DE-OS 27 44 313 bekannt, die Hydrolyse des Methylformiates in Gegenwart von etwa stöchiometrischen Mengen einer Base wie beispielsweise 1-n-Pentylimidazol vorzunehmen. Hierbei entsteht ein Addukt aus Base und Ameisensäure, von welchem sich die übrigen Reaktionspartner (Methylformiat, Methanol, Wasser) destillativ leicht abtrennen lassen, wonach man die wasserfreie oder weitgehend wasserfreie Ameisensäure in einem weiteren Verfahrensschritt von dem Addukt abdestilliert. Da das Addukt verhältnismäßig stabil ist, bedarf es zu seiner Spaltung relativ scharfer Destillationsbedingungen. Wendet man hierzu höhere Temperaturen an, so bilden sich unerwünschte Zersetzungsprodukte, welche die Ameisensäure auch in Spuren verfärben, und wenn man die Spaltung bei stark vermindertem Druck vornimmt, verläuft die Destillation zu langsam.

Ferner ist es bekannt (DE-OS 25 45 658), Carbonsäuren, darunter Ameisensäure, mit Carbonsäureamiden wie N-Di-n-butylformamid aus ihren wäßrigen Lösungen zu extrahieren und die Säure sodann destillativ von der Extraktphase abzutrennen. Hierbei handelt es sich jedoch um eine flüssig-flüssig-Extraktion, die vorzugsweise bei relativ niederen Temperaturen vorgenommen wird und sich deshalb wegen der erforderlichen Zwischenkühlung nicht harmonisch in einen Gesamtprozeß zur Herstellung von Ameisensäure durch Hydrolyse von Methylformiat einfügen läßt.

Nach dem Verfahren der DE-OS 25 45 730 schließlich gewinnt man Ameisensäure aus ihren wäßrigen Lösungen durch Extraktivdestillation mit N-Formylmorpholin und anschließende destillative Zerlegung der Extraktphase in Ameisensäure und das Extraktionsmittel. Da man nach diesem Verfahren Wasser und Ameisensäure verdampfen muß, eignet es sich ebenfalls nicht zur wirtschaftlichen Gewinnung der Ameisensäure aus Methylformiat.

Der vorliegenden Erfindung lag daher die Gewinnung wasserfreier oder weitgehend wasserfreier Ameisensäure aus Methylformiat auf einem insgesamt technisch befriedigendem Wege als Aufgabe zugrunde.

Es wurde gefunden, daß man wasserfreie oder weitgehend wasserfreie Ameisensäure durch Hydrolyse von Methylformiat, erhält, wenn man das Verfahren in einer Kolonne ausführt, wobei man

a) die Hydrolyse im Mittelteil der Kolonne im Gegenstrom von Wasser und Methylformiat vornimmt,

b) die gebildete Ameisensäure im unteren Teil der Kolonne mittels eines Carbonsäureamids der allgemeinen Formel I

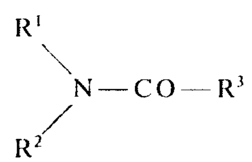

I

in der $R^1$ und $R^2$ Alkylgruppen, die auch zu einem 5- oder 6gliedrigen Ring verbunden sein können, oder die Cyclohexylgruppe und $R^3$ Wasserstoff oder einen $C_1-C_4$-Alkylrest bedeuten, wobei die Summe der C-Atome in den Resten $R^1-R^3$ 7–14 beträgt, extrahiert, wobei man das Carbonsäureamid am unteren Ende des Mittelteils der Kolonne zugibt,

c) die hauptsächlich aus Ameisensäure und dem Carbonsäureamid bestehende Extraktphase im unteren Teil der Kolonne destillativ entwässert oder weitgehend entwässert,

d) im oberen Teil der Kolonne das Methanol sowie nicht umgesetztes Methylformiat durch fraktionierte Destillation abtrennt und

e) aus der wasserfreien oder weitgehend wasserfreien Extraktphase die reine Ameisensäure bzw. eine konzentrierte wäßrige Ameisensäure in einer zweiten Kolonne von dem Carbonsäureamid abdestilliert.

Eine sehr zweckmäßige und energiesparende Ausführungsform dieses Verfaherns besteht darin, daß man in einem zusätzlichen Verfahrensschritt

f) die wäßrige Ameisensäure, die sich im unteren Teil des Mittelteils der Kolonne unterhalb der Zulaufstelle des Methylformiates in flüssiger Form ansammelt, gänzlich oder größtenteils abzieht und einer getrennten Extraktionskolonne zuführt, wo sie mittels des Carbonsäureamids I nach den Methoden der flüssig-flüssig-Extraktion zum Teil entwässert wird, wonach man das hierbei als Raffinatphase anfallende Wasser zweckmäßigerweise wieder in den oberen Teil des Mittelteils der Kolonne zurückführt und die Extraktphase, die aus der Ameisensäure, dem Extraktionsmittel und einer Teilmenge des der Extraktionskolonne zugeführten Wassers besteht, unterhalb der Entnahmestelle der wäßrigen Ameisensäure in den unteren Teil des Mittelteils der Kolonne zurückführt.

Beide Verfahrensweisen als Bestandteil der Gesamtsynthese der Ameisensäure seien anhand der Zeichnungen erläutert.

Wie man aus Figur 1 erkennt, bildet das durch die Merkmale (a) — (e) gekennzeichnete erfindungsgemäße Grundverfahren, welches die kombinierte Hydrolyse/Extraktions/Fraktionierkolonne (1) und die Ameisensäurekolonne (2) umfaßt, zusammen mit der Methylformiatsynthese im Reaktor (3) einen geschlossenen Verbund, in den lediglich Wasser und Kohlenmonoxid eingeführt werden und aus welchem lediglich reine oder hochkonzentrierte Ameisensäure entnommen wird. Selbstverständlich findet auch hier ein gewisser Verbrauch an Methanol und dem Carbonsäureamid I statt, wie sie auch in der Ameisensäurekolonne geringe Mengen an Rückstand bilden, jedoch kann dies unberücksichtigt bleiben, da es das Prinzip des Verfahrens nicht berührt.

Herzstück des Verfahrens ist die kombinierte Hydrolyse/Extraktions/Fraktionier-Kolonne (1). Diese Kolonne besteht entsprechend ihren Funktionen aus dem unteren Teil (4, im folgenden als Extraktionsteil bezeichnet), dem mittleren Teil (5, »Hydrolyseteil«) und dem oberen Teil (6, »Fraktionierteil«).

Vorzugsweise wird die Kolonne bei Normaldruck betrieben, wobei man es so einrichtet, daß die Temperatur im Kolonnensumpf 150 — 175° C, am unteren Ende des Hydrolyseteils 90 — 105° C, am oberen Ende des Hydrolyseteils 75 — 90° C und am Kopf 31 — 35° C beträgt. Arbeitet man bei Unterdruck (etwa bis zu 700 mbar absolut) oder bei Überdruck (etwa bis zu 1,5 bar), so ändern sich diese Temperaturen nach bekannten Gesetzmäßigkeiten entsprechend.

Für den Verfahrensschritt (a), die Hydrolyse im Gegenstromverfahren, leitet man das Wasser in den oberen und das Methylformiat in den unteren Bereich des Hydrolyseteils der Kolonne. Die Bauart des Hydrolyseteils (5) der Kolonne soll so beschaffen sein, daß die für die Hydrolyse erforderliche Verweilzeit, die etwa 1 — 30 Minuten beträgt, gewährleistet wird.

Theoretisch würde im Hydrolyseteil 1 Boden genügen, jedoch würde sich hier nur ein unbefriedigendes Gleichgewicht zugunsten der Ameisensäure einstellen. Dieses Gleichgewicht verbessert sich nach dem Massenwirkungsgesetz, wie allgemein bekannt ist, mit zunehmender Bodenzahl, der jedoch technisch-wirtschaftliche Grenzen gesetzt sind. Befriedigende Verfahrenserfolge erzielt man bereits mit 6 Böden, wogegen mehr als 20 Böden keinen nennenswerten Vorteil mehr bringen. Bevorzugt wird daher eine Bodenzahl von 8 — 15.

Entsprechend der Funktion des Hydrolyseteils der Kolonne, bestimmte Verweilzeiten einzuhalten, wird für die Böden das Bauprinzip der Glockenböden bevorzugt, wobei es auf die spezielle Bauart der in zahlreichen Varianten technisch gebräuchlichen Glockenböden nicht ankommt. Unter diesen Bauarten sind jedoch solche besonders zu empfehlen, die einen hohen Flüssigkeitsstand über der Bodenplatte gestatten. Auch mit Ventilböden lassen sich gute Verfahrensergebnisse erzielen. Siebböden können, besonders in größerer Anzahl, ebenfalls verwendet werden, sind im allgemeinen aber weniger gut geeignet.

Will man wasserfreie Ameisensäure herstellen, so führt man der Reaktion Wasser und Methylformiat im Molverhältnis von etwa 0,90 : 1 bis 0,95 : 1 zu. Der Methylformiat-Überschuß ist deshalb erforderlich, weil stets ein Teil des Methylformiats in den Methanol-Seitenabzug gelangt und der Umsetzung somit entzogen wird. Im Falle wasserhaltiger Ameisensäure erhöht sich die Wassermenge entsprechend. Für die Hydrolyse im stationären Betrieb richtet man es jedoch so ein, daß das Molverhältnis von Wasser zu Methylformiat im Hydrolyseteil 30 : 1 bis 100 : 1 beträgt, d. h. zu Beginn der Reaktion ist zur Füllung des Hydrolyseteils zunächst eine entsprechende Menge überschüssiges Wasser zuzuleiten.

Die Hydrolyse verläuft infolge der gebildeten starken Ameisensäure autokatalytisch, jedoch kann man auch katalytische Mengen einer starken Säure wie p-Toluolsulfonsäure oder Schwefelsäure mitverwenden. Die hierdurch erzielbare höhere Hydrolysegeschwindigkeit ist indes gegen den Nachteil abzuwägen, daß diese Säuren mit dem Carbonsäureamid I reagieren, so daß man Verluste an I oder zumindest Regenerierungskosten in Kauf nehmen muß.

Der über dem Hydrolyseteil (5) angeordnete Fraktionierteil (6) gemäß Verfahrensmerkmal (d) hat die Aufgabe der Fraktionierung von Methanol und Methylformiat, wobei das Methanol als flüssiger Seitenabzug weitgehend abgetrennt und Methylformiat am Kopf der Kolonne dampfförmig oder flüssig entnommen wird. Eine praktisch quantitative Abtrennung des Methanols ist hierbei mittels einer theoretischen Bodenzahl ab 15 möglich, jedoch enthält das Methanol stets noch etwas Methylformiat. Da das Methanol in die Synthesestufe zurückgeführt wird und das Methylformiat hier nicht stört, ist es insgesamt wirtschaftlicher, Gemische aus Methanol und etwa 5–20 Gew.-% Methylformiat abzutrennen. Hierfür reicht ein Fraktionierteil mit 10–25 theoretischen Böden aus.

Die Bauart des Fraktionierteils kann beliebig sein, d. h. dieser Teil kann als Glockenboden-, Ventilboden-, Siebboden- oder Füllkörperkolonne ausgebildet sein.

Aufgabe des Extraktionsteils (4) der Kolonne ist es, die Ameisensäure gemäß Verfahrensmerkmal (b) aus dem Reaktionsgemisch zu extrahieren und gemäß Verfahrensmerkmal (c) gleichzeitig zu entwässern oder weitgehend zu entwässern, indem das Wasser in dem Hydrolyseteil der Kolonne zurückgetrieben wird. Für eine vollständige Entwässerung sind hierfür etwa 10 theoretische Böden erforderlich. Diese Bodenzahl ermäßigt sich auf etwa 5–8, wenn man nur eine 85–95gew.-%ige wäßrige Säure, wie es den technischen und wirtschaftlichen Erfordernissen meistens entspricht, gewinnen will. Für die Art der Kolonnenausführung gilt das gleiche wie für den Fraktionierteil (6).

Als Extraktionsmittel I gemäß Verfahrensmerkmal (b) eignen sich vornehmlich solche definitionsgemäßen Carbonsäureamide, in denen $R^1$ und $R^2$ gleiche Alkylgruppen mit 3–6 C-Atomen oder die Cyclohexylgruppe, und in denen $R^3$ Wasserstoff sowie daneben die Methyl- oder Äthylgruppe bedeuten.

Derartige Carbonsäureamide sind beispielsweise:

Di-n-butylformamid,
Di-n-pentylformamid,
Di-iso-pentylformamid,
Dicyclohexylformamid,
Di-n-butylacetamid,
Di-n-butylpropionamid.

Als besonders gut geeignet hat sich hierunter das Di-n-butylformamid erwiesen.

Da die Carbonsäureamide I als schwach basische Substanzen Wasserstoffbrücken-Addukte mit der Ameisensäure bilden, verwendet man sie zweckmäßigerweise in der Menge, daß sie innerhalb ihres Kreislaufes im Hydrolyseteil (5) der Kolonne (1) in mindestens äquimolarer Menge, bezogen auf die zu extrahierende Ameisensäure, zur Verfügung stehen. Bevorzugt wird jedoch ein Überschuß von 0,1–1 Mol. Allerdings kann auch ein Unterschuß (bis zu 0,5 Mol I pro Mol der zu extrahierenden Ameisensäure) verwendet werden, weil das Carbonsäureamid auch größere als stöchiometrische Mengen an Ameisensäure zu extrahieren vermag.

Der Verfahrensschritt (e) dient zur Vervollständigung der erfindungsgemäßen Lehre, trägt jedoch zum Wesen der Erfindung nichts bei. Dieser Schritt kann daher wie üblich vorgenommen werden, so daß sich nähere Ausführungen hierüber erübrigen. Man erhält bei dieser Destillation die Ameisensäure mit dem Wassergehalt, welcher dem Wassergehalt des zugeführten Carbonsäureamid/Ameisensäure/Wasser-Gemisches entspricht.

Die das zusätzliche Verfahrensmerkmal (f) betreffende zweckmäßige Ausführungsform des erfindungsgemäßen Verfahrens beruht auf der allgemeinen technischen Aufgabe, den Energiebedarf eines großtechnisch ausgeübten Verfahrens soweit wie möglich zu senken, auch wenn hierdurch zunächst höhere Investitionskosten entstehen.

Im vorliegenden Fall nun dient ein erheblicher Teil der Energie dazu, das Wasser im Extraktionsteil der Kolonne zur Gewinnung des wasserfreien oder weitgehend wasserfreien Gemisches aus der Ameisensäure und dem Extraktionsmittel I zu verdampfen und dadurch wieder in den Hydrolyseteil zurückzutreiben. Es bestand daher die spezielle Aufgabe, diesen Energiebedarf zu senken.

Diese Aufgabe wurde dadurch gelöst, daß man die flüssige wäßrige Ameisensäure, die sich im unteren Teil des Hydrolyseteils der Kolonne ansammelt, nach den Methoden der flüssig-flüssig-Extraktion teilweise entwässert, bevor sie in den Extraktionsteil der Kolonne gelangt. Dies bedeutet, daß die in den Extraktionsteil der Kolonne gelangende wäßrige Ameisensäure bereits wasserärmer ist und daß sich damit die zur Verdampfung des Wassers erforderliche Energie entsprechend ermäßigt.

Figur 2 veranschaulicht die apparative Ausgestaltung dieser Verfahrensweise, die sich vom Grundverfahren gemäß Figur 1 im wesentlichen nur durch die Verwendung der zusätzlichen Extraktionskolonne (7) unterscheidet.

Die wäßrige Ameisensäure wird aus dem Hydrolyseteil (5) der Kolonne mindestens einen Boden unterhalb des Zulaufs des Methylformiates entnommen und in die Extraktionskolonne (7) eingeführt, wo sie im Gegenstrom mit dem Extraktionsmittel I extrahiert wird. Das Extraktionsmittel stammt hierbei, wie es sich zur Aufrechterhaltung eines geschlossenen Extraktionsmittel-Kreislaufes von selbst versteht, aus der Destillationskolonne (2). In (7) fällt eine Extraktphase an, welche praktisch die gesamte Ameisensäure, das gesamte Extraktionsmittel und noch einen Teil des Wassers enthält. Diese wasserarme Extraktphase wird sodann in den Hydrolyseteil der Kolonne zurückgegeben, und zwar mindestens einen Boden unterhalb der Entnahmestelle der wäßrigen Ameisensäure. Die Raffinatphase von (7) besteht praktisch nur aus Wasser und wird zweckmäßigerweise anstelle der entsprechenden Menge Frischwasser wieder in den Hydrolyseteil (5) der Kolonne zurückgeführt.

Die Extraktionskolonne (7) kann von beliebiger Bauart sein und soll vorzugsweise 5–12 theoretische Trennstufen haben. Die Extraktion selber

erfolgt in an sich bekannter Weise und bedarf daher keiner weiteren Erläuterungen.

Verglichen mit dem Energiebedarf für das Verfahren gemäß den Merkmalen (a)–(e) beträgt die Ersparnis durch die zusätzliche Ausgestaltung des Verfahrens gemäß Merkmal (f) etwa 40%, ein Wert, welcher den zusätzlichen Investitionsbedarf in aller Regel rechtfertigen dürfte.

Das erfindungsgemäße Verfahren gemäß den Merkmalen (a)–(e) ist verfahrenstechnisch deshalb bemerkenswert, weil es, abgesehen von der Ameisensäurekolonne (2) auf einfache und technisch elegante Weise in einer einzigen Apparatur, nämlich der Kolonne 1 mit ihren drei Teilen (4), (5) und (6) vorgenommen wird, obwohl diese Kolonne die verschiedenartigsten Funktionen erfüllen muß. Die hierdurch bedingten Vorteile sind auch in der besonderen Ausführungsform gemäß dem Merkmal (f) gegeben, obwohl hier eine zusätzliche Extraktionskolonne erforderlich ist.

Beispiel 1

Eine Versuchskolonne von 400 cm Höhe und 5 cm Durchmesser, deren unterer Teil (Extraktionsteil) als Füllkörperkolonne mit 12 theoretischen Böden, deren mittlerer Teil (Hydrolyseteil) als Glockenbodenkolonne mit 12 Böden und deren oberer Teil (Fraktionierteil) wiederum als Füllkörperkolonne mit 21 theoretischen Böden ausgestaltet war, wurde unter Normaldruck nach Erreichen des stationären Betriebes auf Höhe des 12. Bodens des Hydrolyseteils stündlich mit 180 g (10 Mol) Wasser von 25°C und auf Höhe des 1. Bodens dieses Teils stündlich mit 500 g (8,3 Mol) frischem Methylformiat beschickt.

Auf Höhe des 14. Bodens des Fraktionierteils wurden bei 58°C stündlich 251 g Methanol und 29 g Methylformiat entnommen. Das restliche Methylformiat (1300 g pro Stunde) sowie etwas Methanol wurden nach Kondensation zusammen mit dem frischen Methylformiat in die Kolonne zurückgeführt. Auf Höhe des 1. Bodens des Hydrolyseteils, an welchem eine Temperatur von 90°C herrschte, wurden stündlich 940 g (6 Mol) Di-n-butylformamid zugegeben.

Am Kolonnensumpf (170°C) fiel stündlich ein Gemisch aus 940 g des Formamids, 362 g (7,9 Mol) Ameisensäure und 40 g Wasser an. Die Destillation dieses Gemisches in einer nachgeschalteten Kolonne lieferte bei 70 mbar absolut stündlich rund 400 g 90gew.-%ige wäßrige Ameisensäure sowie praktisch das gesamte Formamid, welches in den Kreislauf zurückgegeben wurde.

Die mittlere Verweilzeit aller Reaktionsteilnehmer betrug etwa 15 Minuten, wovon etwa 13 Minuten auf die Verweilzeit im Hydrolyseteil der Kolonne entfielen. Die Wärmezufuhr erfolgte über einen Dünnschichtverdampfer und war so bemessen, daß sich am Kolonnenkopf ein Rücklaufverhältnis von 3–3,5 einstellte.

Durch Erhöhung der Energiezufuhr entsprechend einem Rücklaufverhältnis von 4–4,5 und gleichzeitiger Verminderung der Wasserzufuhr wurde die Extraktphase wirkungsvoller entwässert, so daß hierbei eine 95gew.-%ige Säure gewonnen wurde.

Beispiel 2

Eine Versuchskolonne von 450 cm Höhe und 5 cm Durchmesser, deren unterer Teil (Extraktionsteil) als Füllkörperkolonne mit 8 theoretischen Böden, deren mittlerer Teil (Hydrolyseteil) als Glockenbodenkolonne mit 25 Böden und deren oberer Teil (Fraktionierteil) wiederum als Füllkörperkolonne mit 22 theoretischen Böden ausgestaltet war, wurde unter Normaldruck nach Erreichen des stationären Betriebes auf Höhe des 25. Bodens des Hydrolyseteils stündlich mit 145 g frischem Wasser und 1170 g rückgeführtem Wasser, welches aus der weiter unten beschriebenen flüssig-flüssig-Extraktion stammte, und auf Höhe des 6. Bodens des Hydrolyseteils mit 454 g frischem Methylformiat sowie mit 1364 g eines Methylformiates beschickt, welches vom Fraktionierteil der Kolonne stammte und 60 g Methanol enthielt.

Auf Höhe des 15. Bodens des Fraktionierteils wurde bei 58°C stündlich ein Gemisch aus 228 g Methanol und 26 g Methylformiat entnommen, welches in die Synthesestufe zurückgeführt wurde.

Auf Höhe des 2. Glockenbodens des Hydrolyseteils der Kolonne wurden stündlich 2000 g wäßrige Ameisensäure abgezogen, welche dem Kopf einer Drehscheiben-Extraktionskolonne, die 7 theoretische Trennstufen hatte, zugeführt wurden. Diese wäßrige Säure wurde bei 75°C im Gegenstrom mit 2000 g/h Di-n-butylformamid extrahiert. Das bei der Extraktion als Raffinatphase anfallende Wasser wurde, wie oben bereits erwähnt, zusammen mit Frischwasser in den Hydrolyseteil der Gesamtkolonne zurückgegeben und die Raffinatphase wurde ebenfalls in den Hydrolyseteil zurückgeführt, und zwar auf Höhe des ersten Bodens.

Im Kolonnensumpf des Extraktionsteils (170°C) fiel stündlich ein Gemisch aus 350 g Ameisensäure, 17 g Wasser und 2000 g des Extraktionsmittels an, welches in der Ameisensäurekolonne auf die in Beispiel 1 beschriebene Weise auf 95%ige Ameisensäure aufgearbeitet wurde. Das hierbei im Sumpf anfallende Extraktionsmittel wurde sodann in die Extraktionskolonne zurückgeführt.

Verglichen mit der Arbeitsweise von Beispiel 1 betrug die Energieersparnis etwa 40%.

**Patentansprüche**

1. Verfahren zur Gewinnung von wasserfreier oder weitgehend wasserfreier Ameisensäure durch Hydrolyse von Methylformiat, dadurch

gekennzeichnet, daß man das Verfahren in einer Kolonne ausführt, wobei man

a) die Hydrolyse im Mittelteil der Kolonne im Gegenstrom von Wasser und Methylformiat vornimmt,
b) die gebildete Ameisensäure im unteren Teil der Kolonne mittels eines Carbonsäureamids der allgemeinen Formel I

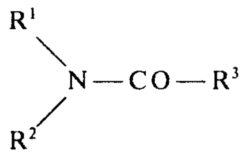

I

in der $R^1$ und $R^2$ Alkylgruppen, die auch zu einem 5- oder 6gliedrigen Ring verbunden sein können, oder die Cyclohexylgruppe und $R^3$ Wasserstoff oder einen $C_1-C_4$-Alkylrest bedeuten, wobei die Summe der C-Atome in den Resten $R^1-R^3$ 7–14 beträgt, extrahiert, wobei man das Carbonsäureamid am unteren Ende des Mittelteils der Kolonne zugibt,
c) die hauptsächlich aus Ameisensäure und dem Carbonsäureamid bestehende Extraktphase im unteren Teil der Kolonne destillativ entwässert oder weitgehend entwässert,
d) im oberen Teil der Kolonne das Methanol sowie nicht umgesetztes Methylformiat durch fraktionierte Destillation abtrennt und
e) aus der wasserfreien oder weitgehend wasserfreien Extraktphase die reine Ameisensäure bzw. eine konzentrierte wäßrige Ameisensäure in einer zweiten Kolonne von dem Carbonsäureamid abdestilliert.

2. Verfahren nach Anspruch 1, dadurch gekennzeichnet, daß man in einem zusätzlichen Verfahrensschritt

f) die wäßrige Ameisensäure, die sich im unteren Teil des Mittelteils der Kolonne unterhalb der Zulaufstelle des Methylformiates in flüssiger Form ansammelt, gänzlich oder größtenteils abzieht und einer getrennten Extraktionskolonne zuführt, wo sie mittels des Carbonsäureamids I nach den Methoden der flüssig-flüssig-Extraktion zum Teil entwässert wird, wonach man das hierbei als Raffinatphase anfallende Wasser zweckmäßigerweise wieder in den oberen Teil des Mittelteils der Kolonne zurückführt und die Extraktphase, die aus der Ameisensäure, dem Extraktionsmittel und einer Teilmenge des der Extraktionskolonne zugeführten Wassers besteht, unterhalb der Entnahmestelle der wäßrigen Ameisensäure in den unteren Teil des Mittelteils der Kolonne zurückführt.

3. Verfahren nach den Ansprüchen 1 und 2, dadurch gekennzeichnet, daß der untere Kolonnenteil 5–10, der mittlere 1–20 und der obere 10–25 theoretische Böden umfaßt.

4. Verfahren nach den Ansprüchen 1 bis 3, dadurch gekennzeichnet, daß der Mittelteil der Kolonne, in welchem die Hydrolyse stattfindet, als Glockenbodenkolonne ausgebildet ist.

5. Verfahren nach den Ansprüchen 1 bis 4, dadurch gekennzeichnet, daß man als Extraktionsmittel Di-n-butylformamid verwendet.

## Claims

1. A process for producing anhydrous or substantially anhydrous formic acid by hydrolysis of methyl formate, characterized in that the process is carried out in a column

a) the hydrolysis being carried out in the middle section of the column, with water and methyl formate in counter-current,
b) the resulting formic acid being extracted, in the lower section of the column, by means of a carboxylic acid amide of the general formula I

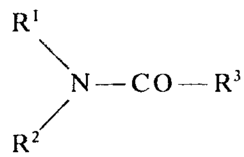

I

where $R^1$ and $R^2$ are alkyl, which may also be linked to form a 5-membered or 6-membered ring, or are cyclohexyl, and $R^3$ is hydrogen or $C_1-C_4$-alkyl, the sum of the carbon atoms in radicals $R^1$, $R^2$ and $R^3$ being from 7 to 14, the carboxylic acid amide being fed into the lower end of the middle section of the column,
c) the extract phase, consisting in the main of formic acid and the carboxylic acid amide, being distillatively dehydrated, or substantially dehydrated, in the lower section of the column,
d) the methanol and unreacted methyl formate being removed by fractional distillation in the upper section of the column, and
e) the pure formic acid or concentrated aqueous formic acid being distilled from the anhydrous or substantially anhydrous extract phase in a second column, leaving the carboxylic acid amide.

2. A process as claimed in claim 1, characterized in that in an additional procedural step

f) the aqueous formic acid collecting in liqued form in the lower zone of the middle section of the column below the methyl formate feed point is completely or largely withdrawn and supplied to a separate extraction column in which it is partly dehydrated by means of carboxylic acid amide I by the method of liquid-liquid extraction, whereupon the water thus obtained as the raffinate phase is advantageously returned

to the upper zone of the middle section of the column, and the extract phase comprosing the formic acid, the extractant and some of the water supplied to the extraction column is returned to the lower zone of the middle section of the column below the outlet for aqueous formic acid.

3. A process as claimed in claims 1 and 2, characterized in that the lower column section comprises 5 to 10 theoretical trays, the middle section comprises 1 to 20 theoretical trays and the upper section comprises 10 to 25 theoretical trays.

4. A process as claimed in claims 1 to 3, characterized in that the middle section of the column, in which the hydrolysis takes place, is a bubble-cap tray column.

5. A process as claimed in claims 1 to 4, characterized in that di-n-butylformamide is used as the extractant.

**Revendications**

1. Procédé pour préparer l'acide formique anhydre ou pratiquement anhydre par hydrolyse du formiate de méthyle, caractérisé en ce que l'on opère dans une colonne dans les conditions suivantes:

a) on effectue l'hydrolyse dans la partie médiane de la colonne dans un contre-courant d'eau et de formiate de méthyle,
b) on extrait l'acide formique formé dans la partie inférieure de la colonne à l'aide d'un carboxamide de formule générale I

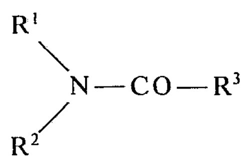

I

dans laquelle $R^1$ et $R^2$ représentent des groupes alkyle qui peuvent également être combinés sous forme d'un cycle à 5 ou 6 chaînons ou le groupe cyclohexyle et $R^3$ représente l'hydrogène ou un groupe alkyle en C1–C4, la somme des atomes de carbone des groupes $R^1$ à $R^3$ allant de 7 à 14, en introduisant le carboxamide à l'extrémité inférieure de la partie médiane de la colonne,
c) on déshydrate totalement ou pratiquement totalement la phase extrait consistant principalement en acide formique et le carboxamide par distillation dans la partie inférieure de la colonne,
d) on sépare le méthanol et le formiate de méthyle non converti par distillation fractionnée dans la partie supérieure de la colonne et
e) on soumet la phase extrait anhydre ou pratiquement anhydre à une distillation dans une deuxième colonne au cours de laquelle on sépare l'acide formique pur ou un acide formique aqueux concentré du carboxamide.

2. Procédé selon la revendication 1, caractérisé en ce que, dans un stade opératoire supplémentaire:

f) on évacue en totalité ou en plus grande partie l'acide formique aqueux recueilli à l'état liquide dans la partie inférieure de la partie médiane de la colonne au-dessous de l'endroit d'alimentation du formiate de méthyle et on l'envoie dans une colonne d'extraction séparée dans laquelle on le déshydrate en partie à l'aide du carboxamide I par les techniques de l'extraction liquide-liquide après quoi l'eau obtenue dans cette opération en phase raffinat est recyclée avantageusement dans la partie supérieure de la partie médiane de la colonne et la phase extrait, qui consiste en l'acide formique, l'agent d'extraction et une partie de l'eau envoyée à la colonne d'extraction, est recyclée dans la partie inférieure de la partie médiane de la colonne au-dessous de l'endroit de prélèvement de l'acide formique aqueux.

3. Procédé selon les revendications 1 et 2, caractérisé en ce que la partie inférieure de la colonne comprend 5 à 10, la partie moyenne 1 à 20 et la partie supérieure 10 à 25 plateaux théoriques.

4. Procédé selon les revendications 1 à 3, caractérisé en ce que la partie médiane de la colonne dans laquelle l'hydrolyse se produit est aménagée en colonne à plateaux à cloches.

5. Procédé selon les revendications 1 à 4, caractérisé en ce que l'on utilise comme agent d'extraction le di-n-butylformamide.

FIG.1

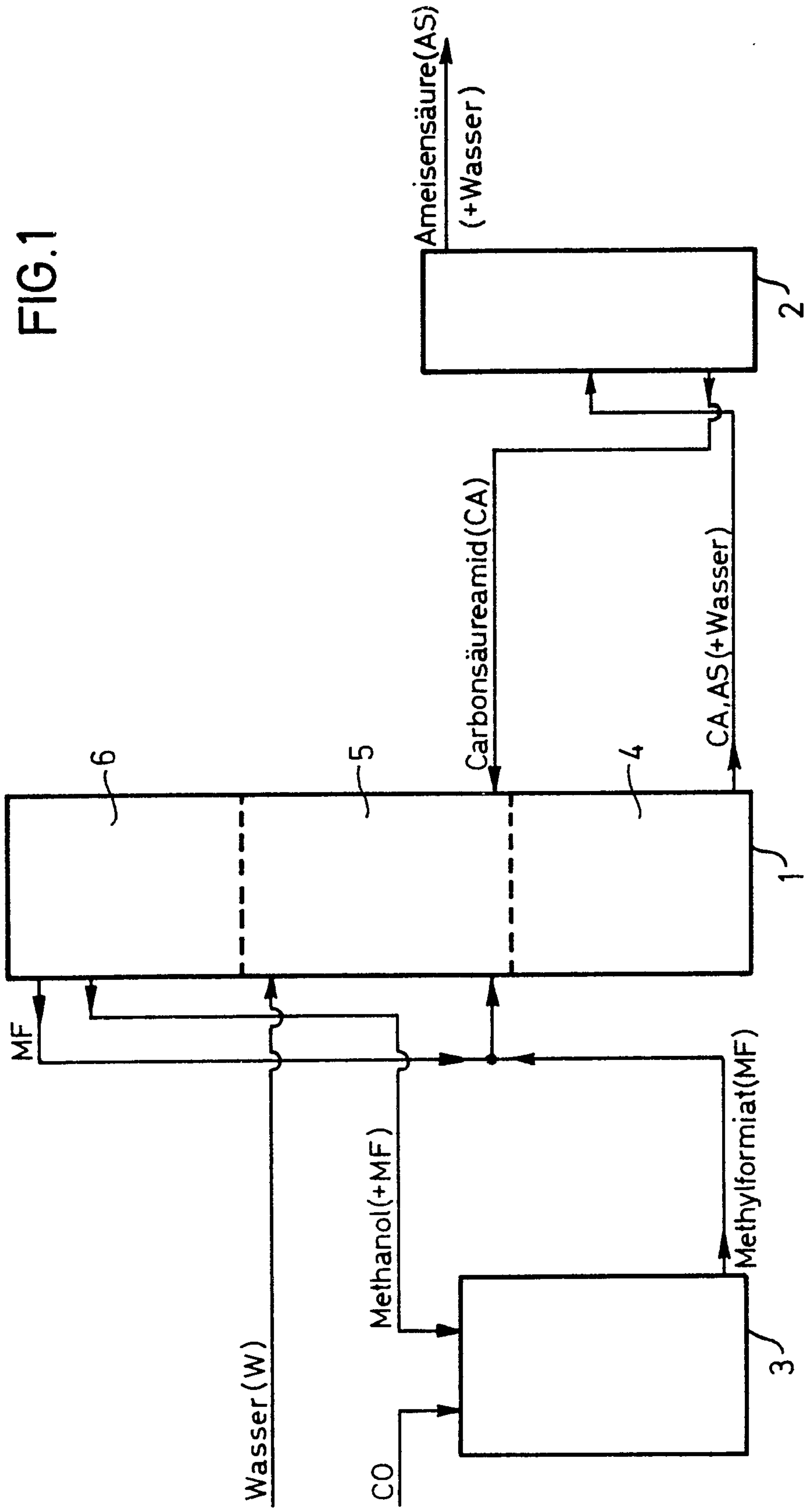
Ameisensäure(AS)
(+Wasser)
2
Carbonsäureamid(CA)
CA,AS(+Wasser)
6
5
4
1
MF
Methanol(+MF)
Methylformiat(MF)
Wasser(W)
CO
3

FIG.2

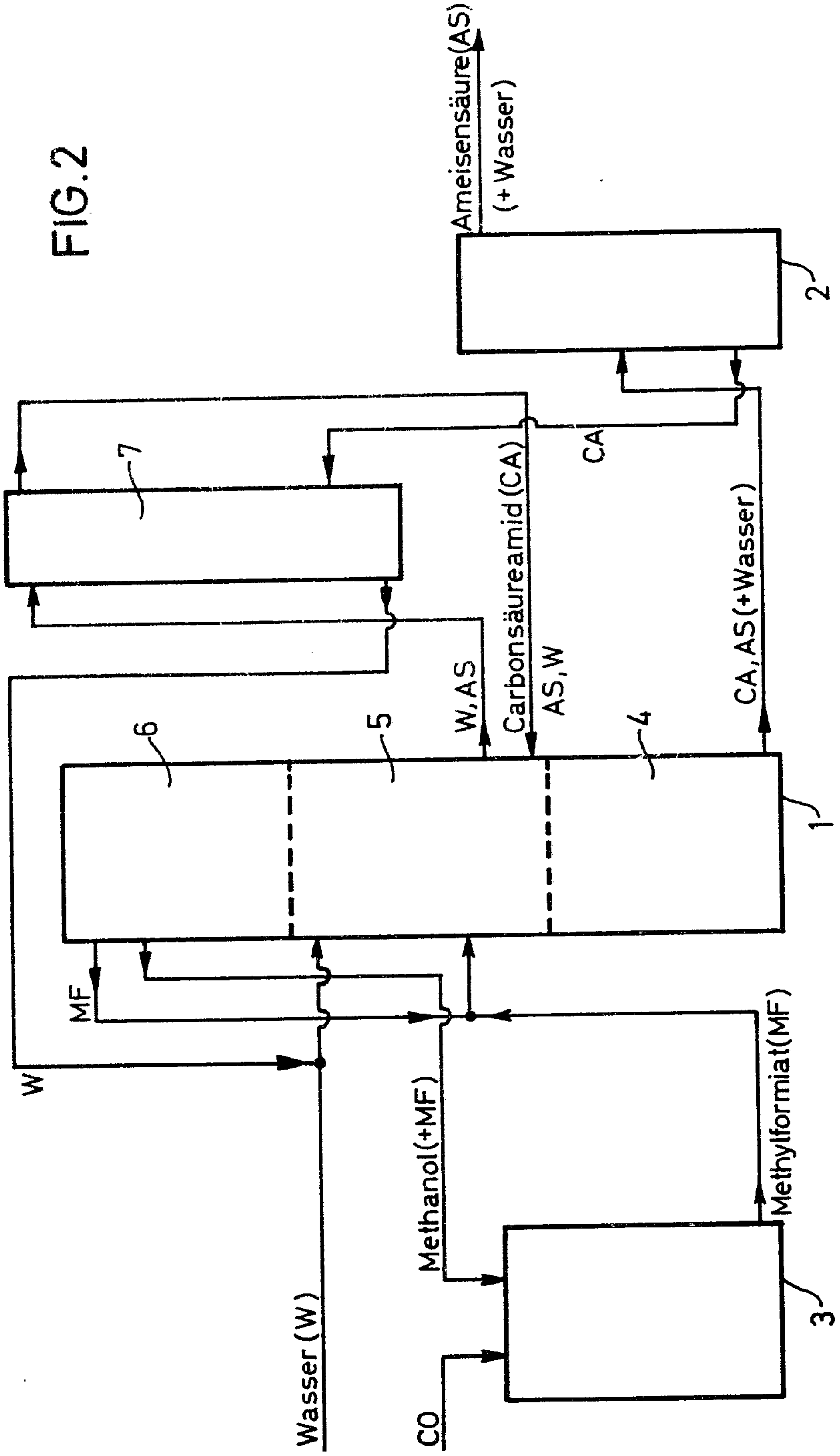
Ameisensäure(AS)
(+Wasser)
2
7
CA
Carbonsäureamid(CA)
CA,AS(+Wasser)
W,AS
AS,W
6
5
4
1
MF
W
Methanol(+MF)
Methylformiat(MF)
3
Wasser (W)
CO